(19)

**Europäisches Patentamt**
**European Patent Office**
**Office européen des brevets**

(11) **EP 2 402 030 A1**

(12) **EUROPEAN PATENT APPLICATION**

(43) Date of publication:
**04.01.2012 Bulletin 2012/01**

(21) Application number: **10168359.7**

(22) Date of filing: **02.07.2010**

(51) Int Cl.:
**A61K 39/35** (2006.01)  **C07K 7/04** (2006.01)
**C07K 7/06** (2006.01)  **C07K 16/16** (2006.01)
**G01N 33/68** (2006.01)  **A01H 5/04** (2006.01)
**A01H 5/06** (2006.01)

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **Charité Universitätsmedizin Berlin
10117 Berlin (DE)**

(72) Inventor: **The designation of the inventor has not yet been filed**

(74) Representative: **Schulz Junghans
Chausseestraße 5
10115 Berlin (DE)**

(54) **Linear IgE peptide epitopes of celery allergen api 1**

(57) Linear polypeptide epitopes of celery allergen api 1.0101 of a sequence length of ten to fifteen amino acids for treatment and prevention of celery allergy are provided. Means and methods for diagnosing celery allergy, and for detecting celery allergens in a sample, are also provided.

EP 2 402 030 A1

## Description

Celery is a frequent cause of food allergy in pollen-sensitized patients and may induce severe allergic reactions. Allergic reactions against celery are of major clinical relevance due to celery's wide presence in convenience food and spice.

Food allergy is currently diagnosed by clinical history and additional tests. For *in vivo* diagnosis the patient is brought into direct contact with the allergenic substance using prick or challenge tests. Investigations of the diagnostic value of the skin prick test, histamine release, and determination of allergen specific IgE (CAP) show a discrepancy between sensitization and the probability of a clinical reaction in patients sensitized to different allgergens. Therefore the double-blind, placebo-controlled food challenge (DBPCFC) remains the gold standard in the diagnosis of food allergies. However, the procedure is time consuming, cost intensive, and not without risk for the patient.

The major allergen of celery is the protein Api g 1.0101 *(Apium graveolens)* which is a Bet v 1 *(Betula verrucosa)* homologue. It belongs to the family of pathogenesis-related plant proteins PR-10. T cell epitopes for Api 1 have been determined (Bohle et al. (2003) Eur. J. Immunol. 33, 3303-3310), however studies showing that IgE responses to Api 1 are abrogated by heating the protein antigen, whereas T cell responses are not (Bohle et al. J. (2006) Allergy Clin. Immunol. 118, 242-249) indicate that IgE recognizes an epitope defined by the tertiary structure of the protein chain, rather than a short linear epitope defined by the peptide amino acid sequence.

Based on this state of the art, it is an objective of the present invention to provide means to diagnose celery allergy in patients, to detect the presence of celery in foods and other products, and to provide means of therapy to patients suffering from celery allergy.

This objective is attained by the subject-matter of the independent claims.

A polypeptide molecule in the context of this description is a linear chain of proteinogenic amino acids linked by amide bonds. Non-peptide linkages may be used to stabilize a polypeptide molecule, conserving the spatial arrangement of side chains making up the recognized epitope. Polypeptide molecules may be covalently modified by acetylation, formylation or benzylation of the terminal amino group, by esterification of the terminal carboxy group or by other common modifications employed in peptide chemistry. The polypeptide side chains, where basic or acidic, may be present in their respective salt form.

Peptide mimotopes may be designed for a particular purpose by addition, deletion or substitution of elected amino acids. Thus, the peptides may be modified for the purposes of ease of conjugation to a carrier. For example, it may be desirable for some chemical conjugation methods to include a terminal cystein. In addition it may be desirable for peptides conjugated to a protein carrier to include a hydrophobic terminus distal from the conjugated terminus of the peptide, such that the free unconjugated end of the peptide remains associated with the surface of the carrier protein, thereby presenting the peptide in a conformation which most closely resembles that of the peptide as found in the context of the whole native molecule.

Polypeptide sequences are given in standard one letter amino acid code (see: Hausmann et al., The Cell: a molecular approach, ISBN 0-87893-214-3) and refer, where not indicated otherwise, to L-amino acids linked by peptide bonds.

The present invention was made in the context of a study to identify specific IgE binding peptides by an array technique. Sera of 21 human patients with positive food challenge to celery as well as sera of 17 healthy individuals were used. All participants underwent skin tests along with determinations of specific IgE. An array of overlapping peptides representing the entire sequence of major celery allergen Api g 1.0101 was synthesized and probed with the participants' sera. Sera of celery allergic patients show binding to three distinct regions of Api g 1.0101. In particular, the region of amino acid 100 to 126 is the most important region for IgE-binding. This region caused a five times higher IgE-binding of celery allergic patients than those of healthy individuals. In particular, one peptide (VLVPTADGGSIC) was recognized by all sera of celery allergic patients. In contrast, no binding to this peptide was detected in sera of the healthy controls.

Hence, the subject-matter of the present invention allows distinguishing between celery allergic and healthy individuals, it provides for a method and means to treat patients suffering from celery allergy and it allows for the detection of IgE specific peptide epitopes that celery allergic patients are prone to react to.

In particular, according to a first aspect of the present invention, a polypeptide molecule is provided for the treatment or prevention of celery allergy, the polypeptide having a sequence length of ten to fifteen amino acids, and an amino acid sequence comprised as a contiguous sequence in any of the following peptide sequences:

SVSAEKIFQGFVIDVDTVLPK (API 1.5-8; SEQ 012),
GDGGPGTLKIITLPDGGPITTMTLRID (API 1.16-21; SEQ 013),
NHVVLVPTADGGSICKTTAIF (API 1.34-37; SEQ 014).

According to a preferred embodiment of this aspect of the invention, the polypeptide has a length of 10 to 12 amino acids. Even more preferred is a dodecapeptide with any of the following sequences:

VLVPTADGGSIC (API 1.35; SEQ 001), AEKIFQGFVIDV (API 1.06; SEQ 002),
IFQGFVIDVDTV (API 1.07; SEQ 003), GFVIDVDTVLPK (API 1.08; SEQ 004),
GDGGPGTLKIIT (API 1.16; SEQ 005), GPGTLKIITLPD (API 1.17; SEQ 006),
TLKIITLPDGGP (API 1.18; SEQ 007), IITLPDGGPITT (API 1.19; SEQ 008),
LPDGGPITTMTL (API 1.20; SEQ 009), GGPITTMTLRID (API 1.21; SEQ 010),
PTADGGSICKTT (API 1.36; SEQ 011); SEQ 001 being the most preferred.

According to a different aspect of the invention, a medical composition comprising polypeptide molecules according to the first aspect of the invention is provided. Such medical composition may comprise only one species of polypeptide molecule, SEQ 001 being the most preferred embodiment. It may, alternatively, comprise a plurality of species as indicated according to the first aspect of the invention.

A medical composition comprising one or several polypeptide species according to this aspect of the invention may include additional components or excipients useful for treating or preventing allergy or atopic reactions. In particular, a pharmaceutical composition according to this aspect of the invention may comprise, for example and without limitation, immune modulators such as toll like receptor 2, 4 or 9 ligands, interferons, interleukins or other factors contributing to specific immunotherapy. Such medical composition may be applied by oral, intravenous, intranodal and other routes.

According to another preferred embodiment of this aspect of the invention, the one or several polypeptide species may be covalently or non-covalently linked to a surface, or to a larger molecular structure such as a dendrimer, polymer -in particular, polyethylene glycol- or other form of presenting antigenic epitopes in a repeated pattern to immune cells. One example of a carrier suited for allergy hyposensitisation is protein D from hemophilus influenzae.

According to yet another preferred embodiment of this aspect of the invention, the one or several polypeptide species may be covalently linked to sugar or fatty acid moieties.

According to a different aspect of the present invention, a method of diagnosing celery allergy in a patient is provided. This method comprises the steps of providing a polypeptide molecule having a sequence length of ten to fifteen amino acids, said polypeptide molecule having an amino acid sequence comprised as a contiguous sequence in SEQ 012, SEQ 013 or SEQ 014, contacting said polypeptide molecule with a patient sample comprising IgE molecules ex-vivo under conditions allowing for the specific binding of IgE molecules to IgE epitopes, and determining whether said polypeptide molecule is specifically bound by an IgE molecule comprised in said patient sample.

According to another preferred embodiment of this aspect of the invention, the polypeptide, or a plurality of polypeptides of the lengths and sequences indicated above, is attached to a surface, a patient sample is brought into contact with said surface, resulting in IgE molecules specific for said polypeptides binding to the polypeptides. Subsequently, any IgE molecules not bound to the polypeptides are washed off, and the binding of IgE molecules is determined. This may be effected preferably either by bringing the surface in contact with a binding agent for IgE molecules, such as an IgE-specific secondary antibody, or by determining the binding event through a change of physical parameters effected by the binding event, e.g. surface plasmon resonance or other methods known in the art.

According to another preferred embodiment of this aspect of the invention, a ligand specific for the constant region of IgE molecules is attached to a surface, and a patient sample is brought into contact with said surface, resulting in any IgE molecules present in the sample becoming attached to the surface. Subsequently, the polypeptide, or a plurality of polypeptides of the lengths and sequences indicated above, is brought into contact with the surface, the polypeptide being modified by a means of detection such as an optically active chemical group or other detectable label.

According to yet a different aspect of the present invention, a means for diagnosing celery allergy is provided, comprising polypeptide molecules having a sequence length of ten to fifteen amino acids, said polypeptide molecules having an amino acid sequence comprised as a contiguous sequence in SEQ 012, SEQ 013 or SEQ 014.

Both the method and the means for diagnosing celery allergy in a sample, according to a preferred embodiment according to the respective aspect of the invention, may employ a polypeptide molecule that has a sequence length of 10 to 12 amino acids, and an amino acid sequence comprised as a contiguous sequence in any of the peptide sequences comprised in the group of sequences denominated as SEQ 001, Seq 002... Seq 011 above. A sequence length of twelve is most preferred.

According to another preferred embodiment of this aspect of the invention, the polypetide molecules are attached to a surface, preferably the surface of an ELISA plate, a surface plasmon chip or a quartz microbalance. Other ways to practice the invention is the attachment of the polypeptide molecules to a glass or plastic slide or bead, a silicon wafer, a magnetic bead, a polystyrene or sepharose bead, a cellulose or polypropylene membrane.

According to yet a different aspect of the invention, a method of detecting the presence of celery allergen api 1 peptide epitopes in a sample is provided, comprising the steps of providing a molecular structure capable of specifically binding a polypeptide molecule comprising an amino acid sequence comprised as a contiguous sequence in SEQ 012, SEQ 013 or SEQ 014, contacting a sample with said molecular structure, and determining whether celery allergen api 1 peptide epitopes have bound to said molecular structure.

Similarly, according to yet a different aspect of the invention, a means for detecting the presence of celery allergen api 1 peptide epitopes in a sample is provided, comprising a molecular structure capable of specifically binding a polypeptide molecule having a sequence length of ten to fifteen amino acids, said polypeptide molecule having an amino acid sequence comprised as a contiguous sequence in SEQ 012, SEQ 013 or SEQ 014.

Both the method and the means for detecting the presence of celery allergen api 1 peptide epitopes in a sample, according to a preferred embodiment according to the respective aspect of the invention, may employ any molecular structure capable of selectively and specifically binding 10 to 15mer polypeptide molecules. Immunoglobuline molecules are an example of such molecular structures. Recombinant fragments of immunoglobulins, monoclonal antibody fragments, and camel antibodies or fragments thereof are examples of molecular structures that can be obtained by methods known in the art for such purpose. IgE variable regions are preferred, but other ligands may be used such as nucleic acid aptamers, protein ligands as derived from phage display selection or other means of evolution based development of highly specific ligands.

Short description of the figures

Fig. 1A    shows an experiment on the influence of peptide length on IgE-binding.

Fig. 1B    shows an experiment on the influence of membrane type and peptide density on IgE-binding.

Fig. 1C    shows an experiment on the influence of blocking reagents upon assay quality.

Fig. 2    shows human IgE antibody response to Api g 1.0101 derived 12-meric peptides arrayed on a cellulose membrane.

Fig. 3    shows a comparison between measured spot signal intensities and IgE antibody response to Api g 1.0101 derived 12-meric peptides arrayed on a cellulose membrane.

**Examples**

The protein sequence of the major allergen in celery Api g 1.0101 (UniprotKB/Swissprot entry P49372) was dissected into sets of overlapping peptides (pepscan). Subsequently, these sequences were synthesized as peptide arrays on cellulose membranes according to SPOT technology. A highly specific secondary anti-human IgE antibody with minimum cross-reactivity towards cellulose membrane-bound peptides was employed.

Synthetic arrays were probed with sera randomly chosen from a well described cohort of subjects, including 21 patients with a defined allergic reaction to celery and 17 healthy individuals. Captured human IgE antibodies were detected by a HRP-labeled secondary antibody and binding was visualized by chemiluminescence.

Clinical profiles of the celery-allergic and healthy subjects

Sera from 21 patients positively tested for native celery root by a skin prick test (SPT) were used for the study. These patients had elevated levels of specific IgE antibodies to celery and a relevant reaction to celery (proven by DBPCFC). Sera from 17 healthy individuals who were negatively tested for native celery root, birch, grass, and mugwort pollen, and showed no adverse reactions to celery, were used as a control.

Quality of membrane bound peptides

To monitor SPOT peptide synthesis quality, a set of 7mer, 12mer and 24mer Api g 1.0101 derived sequences was randomly chosen. 31 peptides were SPOT synthesized on a β-alanine membrane, subsequently cleaved from the membrane via dry state aminolysis and analyzed by HPLC and ESI mass spectrometry. N-CAPE membranes proved to be preferable for probing IgE antibody binding. Thus peptide quality control was repeated with a total of 155 peptides synthesized on N-CAPE membranes. To guarantee peptide cleavage from the N-CAPE membrane, the acid label Fmoc-Rink-linker was used. Peptides were released as amides from the N-CAPE membranes using a two step cleavage protocol.

Importance of peptide length

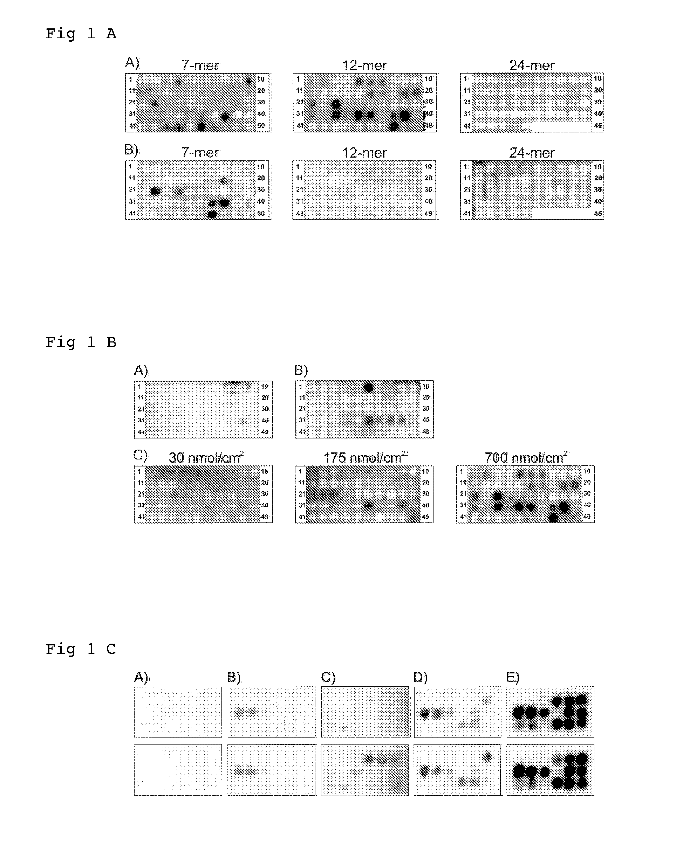

Different lengths of overlapping peptides from the Api g 1.0101 sequence (7-, 12- and 24-meric peptides) were investigated. Peptide arrays were probed with sera of healthy and celery allergic subjects. Fig. 1A shows the influence of peptide lengths on IgE-binding. Overlapping peptides of Api g 1.0101 were synthesized on N-CAPE membranes and (A) incubated with patients sera or (B) with detection antibody alone. IgE-binding was detected using an HRP-labeled anti-IgE antibody. Incubation results with 7-mer, 12-mer and 24-mer peptides are shown.

A reduction of peptide length induces an increase of binding interaction, but correlates with an increase of secondary antibody cross-reactivity (false positives) to the membrane bound peptides. An optimal ratio of sera signal to false positives was observed using arrays of 12-meric peptides, with 10 to 15-mers offering useful results.

Cellulose membrane type and peptide density

Membrane types and membrane functionality influence both the quality of binding assays. Arrays of 12-meric peptides covering the Api g 1.0101 sequence were synthesized on two kinds of membranes: an ester type membrane (b-alanine membrane) and an ether type membrane (N-CAPE membrane). These peptide arrays were probed for human IgE antibody binding, revealing that the N-CAPE membrane are preferred for human IgE antibody sera screening (Fig. 1B lanes (A), (B), (C)). 12-meric peptides of Api g 1.0101 were sythesized (A) on b-alanine membrane and (B) on N-CAPE membrane and incubated with patients sera. (C) Additionally 12-meric peptides were synthesized on N-CAPE membranes with different loadings and incubated with patients sera. Visualization was performed using an HRP-labeled anti IgE-antibody via chemiluminescence.

Compared to the b-alanine membrane, background levels with N-CAPE membranes were lower and signals showed higher signal-to-noise-ratios.

N-CAPE membranes with an amino-functionality in the range of 30 nmol/cm2 to 800 nmol/cm2 were used. Analysis of 12-meric pepscans over the Api g 1.0101 sequence, synthesized on differently loaded N-CAPE membranes, revealed an optimum membrane loading of approximately 640-760 nmol/cm2. Reduction of membrane loading leads to a significant loss in signal intensity and worse signal-to-noise-ratio (Fig. 1B lane(C)).

The mean total IgE concentration was determined for celery allergic individuals at 1.9 $\mu$g/ml, and a celery specific IgE of about 0.009 $\mu$g/ml. In the present study the applied serum was diluted 1:5 prior to array incubation, resulting in a total IgE concentration of approximately 0.4 $\mu$g/ml and a specific IgE concentration below 0.01 $\mu$g/ml.

In serum patient samples, both allergen-specific IgE and IgG are present. IgG concentration is up to 1,000-fold higher compared to IgE concentration, and both immunoglobulins compete for binding to the same peptide. Therefore, IgG binding can hinder detection of IgE. On the other hand the IgE has most likely a higher affinity in comparison to IgG. Therefore high loaded membranes and extended incubation times at low temperature help to avoid such competition effects.

The detection and visualization system

Different anti-IgE antibodies to detect sIgE were used: (i) Two different secondary anti-IgE antibody, labeled with horse radish peroxidise (HRP) (P 0295, Dako, and A 9667, Sigma-Aldrich); (ii) A secondary biotin labeled anti-human IgE antibody (BA-3040, Vector laboratories) in combination with streptavidin-HRP (S 5512, Sigma-Aldrich) adduct; (iii) A sandwich-system comprising a mouse anti-human IgE antibody (IgG type, I 6510, Sigma-Aldrich) and a HRP-labeled anti-mouse antibody (A 5906, Sigma-Aldrich).

To determine the optimal detection/visualization system set of overlapping peptides (12-mers) covering the Api g 1.0101 sequence were synthesized on high-loaded N-CAPE membranes and subsequently probed with sera (diluted 1:5). Detection/visualization was performed using one of the systems described above.

Best results were obtained with two HRP labelled anti-human IgE antibodies: polyclonal anti-human IgE anti-bodies, specific for the $\varepsilon$-heavy chain and both labeled with HRP. These antibodies show low cross-reactivity with membrane bound peptides, and excellent human IgE specificity. Anti-human IgE (HRP) (A 9667, Sigma-Aldrich) was selected for subsequent analyses.

Identification of human-IgE antibody binding peptides

The optimal conditions for screening the sera of 21 celery allergic patients and 17 healthy controls were as follows: (i) replica of a 12-meric pepscan with a shift of three amino acids covering the Api g 1.0101 sequence synthesized on high-loaded N-CAPE membranes (640 nmol/cm2 - 760 nmol/cm2); (ii) prior to treatment with sera, membranes were blocked with the Sigma-Genosys blocking buffer for 2 h at room temperature; (iii) arrays were probed for 16 h at 4°C with diluted sera (1:5) for IgE antibody binding; (iv) the captured human IgE antibodies were detected with the HRP-labeled anti-human IgE antibody (A 9667, Sigma-Aldrich) and visualized by chemiluminescence; (v) spot signal intensities were measured on a LumiImager and processed with the LumiAnalyst software. To check the reproducibility of our screening procedure, three different peptide arrays were incubated and evaluated for each person (allergic and healthy).

Sera binding patterns could be reproduced in repeated incubation experiments. Although inter-membrane absolute spot signal intensities differed, the relative differences between spot signal intensities were nearly the same. This enabled comparison of spot signal intensities from different membranes by applying an additional normalization procedure. We defined an IgE antibody binding spot as positive if its normalized signal intensity (SInorm) was higher than the calculated cut off value of the appropriate membrane.

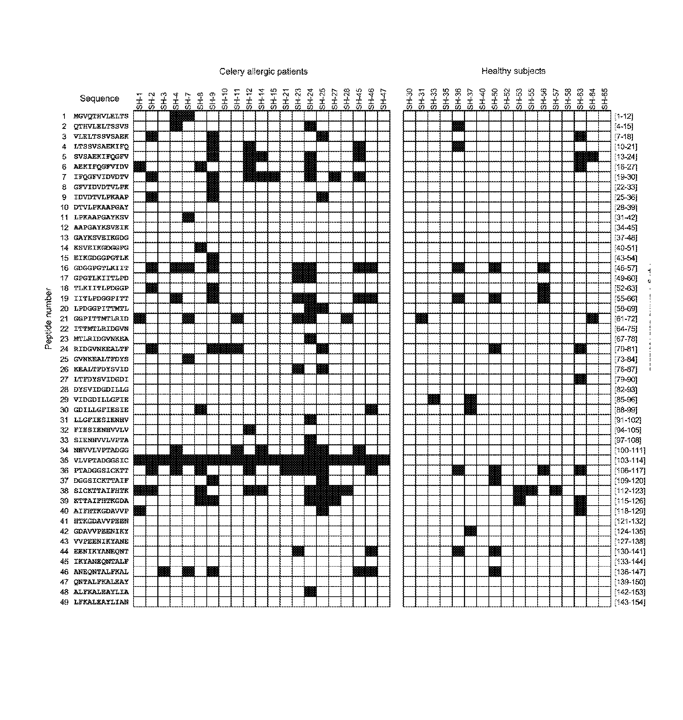

Results of a comprehensive sera screening study are presented in Fig. 2, showing human IgE antibody response to Api g 1.0101 derived 12-meric peptides arrayed on a cellulose membrane and probed for binding by incubation with sera of 21 celery allergic patients and 17 healthy subjects. Subjects recruited for this study denoted as SH and are shown in the upper line divided into celery allergic patients and healthy subjects. The 49 peptide sequences shown on the right column covering the sequence of the main celery allergen Api g 1.0101 with a shift of three amino acids. Captured IgE antibodies were determined by an HRP labeled anti-human IgE antibody and visualize by chemilumiescense. A spot was defined as positive if its normalized signal intensity was higher than the cut off value of the appropriate membrane.

An interaction of serum human IgE antibodies to a peptide derived from the Api g 1.0101 sequence is depicted as a black square, with IgE antibody binding patterns shown for each individual recruited for the study. The binding patterns demonstrate that there are much more IgE antibody binding events in serum from celery allergic patients than from healthy subjects (Fig. 2). The celery allergic cohort revealed 136 positive IgE antibody-binding spots, whereas only 36 positive IgE-binding spots were found in the healthy control group. The sum of positive IgE antibody responses showed significant differences between celery allergic and healthy individuals, ranging from 1 to 18 positive spots per subject in the allergic group and from 0 to 8 in the control group (Wilcoxon, $P < 10^{-3}$). An arithmetic mean of 2.1 positive IgE antibody-binding spots was calculated for healthy individuals and 6.5 positive responses for celery allergic patients. This corresponds to a three-time higher IgE antibody response to Api g 1.0101 derived peptides for celery allergic patients compared to healthy individuals.

Three sequence regions of Api g 1.0101 were predominantly recognized: the first region spans the Api g 1.0101 sequence from amino acids 1 to 30; the middle region from 46 to 72; and the more C-terminal region from amino acid 100 to 126 (Fig. 2). Peptides within the first region were recognized 27-times by sera from celery allergic patients and only 6-times by healthy subjects. Thus, IgE antibody binding preference for this region is 4.5-fold higher in celery allergic patients than in the healthy subjects. The middle region was bound 3-times more frequently by IgE antibody from allergic patients, while the highest IgE antibody binding preference was found for the C-terminal region, which was recognized 5-fold more often by allergic than by healthy individuals. We therefore conclude that the most allergenic regions of the Api g 1.0101 protein are located in the regions between amino acid 1-30 and 100-126, respectively.

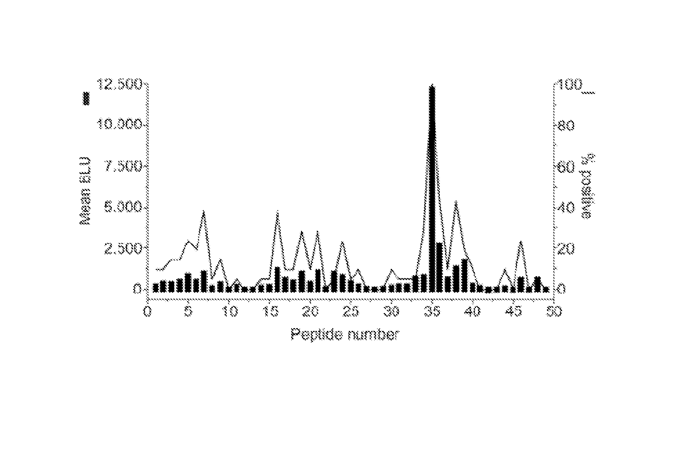

As described above, six IgE antibody-binding events are the average for the celery allergic group. In total, 8 distinct peptides of Api g 1.0101 were recognized by at least 25% of the celery allergic individuals. In detail, 29% responded to peptides 19, 21 and 34; 38% reacted with peptides 7 and 16; 43% responded to peptides 36 and 38; and finally 100% reacted with peptide 35 (peptide sequences are shown on the left in Figure 2). This modus operandi was subsequently applied to all peptide arrays, in addition to measuring the average spot signal intensity in terms of BLU (Fig. 3). Confirming the data in Figure 2, the same three allergenic regions of Api g 1.0101 protein appear in Figure 3: the N-terminal region comprising peptides 1 to 7, the middle region peptides 16 to 21, and the C-terminal region containing peptides 34 to 39. However, the spot signal intensities highlight differences between these regions. Interestingly, the highest spot signal intensities were measured for peptides located in the C-terminal region of Api g 1.0101 (peptides 34 - 39) identified above as the most allergenic region. In contrast, peptides belonging to the middle allergenic region of Api g 1.0101 (peptides 16 to 21) and notably the second most allergenic region in the N-terminus (peptides 1 to 7) show lower signal intensities.

Finally, we searched for sequences binding sera IgE antibodies of at least six celery allergic patients but not reacting with any sera IgE antibody from healthy subjects recruited in this study. Only three sequences were identified: (i) one peptide with the sequence IFQGFVIDVDTV (peptide 7 in Figure 2) belonging to the N-terminal region of Api g 1.0101 and (ii) two peptides located in the C-terminal region with the sequences NHVVLPTADGG (peptide 34) and VLVPTADGGSIC (peptide 35). The latter is the only peptide recognized by the sera from all the celery allergic patients recruited for this study. Even more striking, this peptide had the highest measured spot signal intensity.

These results allow for the conclusion that the major allergic region of the protein Api g 1.0101 is located in the C-terminal region around the sequence VLVPTADGGSIC. Furthermore, we suggest that the allergenic effect of this region is supported by the N-terminal region of Api g 1.0101. This assumption is supported by the positive correlation between the number of recognized peptides in the N-terminal region (peptides 1 - 7) and the C-terminal region (peptides 34 - 39) in sera of celery allergic patients (Spearman rank correlation, r = 0.476, P = 0.025).

**Experimental conditions**

Subjects: Twenty-one adult patients aged between 20 and 49 years with a history of birch or mugwort pollen allergy and of adverse reactions to celery were recruited from the Allergy-Centre-Charité at the Department of Dermatology and Allergology, Charité. Patients were investigated according to the EAACI guidelines using a thorough medical history combined with in vitro and in vivo testing. Exclusion criteria for further analysis were a concomitant severe disease, pregnancy, lactation, the use of antihistamines, b-blockers, ACE inhibitors, neuroleptics, oral steroids or immunosuppressive drugs. A group of skin healthy, non-allergic volunteers (n = 17) served as a control group. To exclude any seasonal effects such as priming or boost by inhaled pollen allergens, all clinical examinations were performed outside the pollen season.

Skin prick test and immunoglobulin E (IgE) determination: Participants received a skin prick test (SPT) with native celery root (prick to prick) and also with commercially available allergens of birch, grass and mugwort pollen (ALK Scherax, Hamburg, Germany). SPTs were performed with a 1 mm lancet (ALK-ABELLO', Hørsholm, Denmark) on the volar surface of the forearm. Histamine dihydrochloride (10 mg/ml) and sodium chloride (0.9% NaCl, ALK Scherax) were used as positive and negative controls, respectively. The reactions were documented after 15 min. The wheal size was measured using the formula: (D+d)/2, where D was the maximum diameter and d its perpendicular diameter. SPTs were considered positive if the diameter of the weal was ≥ 3mm. Serum samples were obtained from all participants at the beginning of the study and were kept at -20°C until use. Specific sensitization to celery was confirmed by the determination of total IgE and specific IgE (celery) using the Phadia CAP System (Uppsala, Sweden).

Double-blind placebo-controlled food challenge: DBPCFC was performed with each patient according to the recommendations of the EAACI and German position papers. Verum and placebo challenges were identical regarding taste, looks, smell, viscosity, texture, structure and volume. For the verum provocation, 50.0 g of fresh finely ground celery root were divided into the following dose steps were given: 5.0 - 10.0 - 15.0 - 20.0 g. The DBPCFCs were titrated with a time interval of 15 min between each provocation dose. Before the administration of the next higher provocation dose, vital parameters such as blood pressure and heart frequency were measured. Monitoring of pulmonary function was performed by measuring the forced expiratory volume in the first second (FEV1) at the beginning of the study, before and after each provocation. Patients with highly unstable conditions (FEV1 below 80% of predicted or the patient's best immediately before the provocation) were excluded from the DBPCFC because of the higher risk for the patient and the enhanced likelihood of false positive responses. Changes in skin status of patients with atopic dermatitis (AD) were assessed by using the Severity Scoring of Atopic Dermatitis (SCORAD).

Functionalization of cellulose membrane: According to the standard conditions for preparing b-alanine functionlized membrane a sheet of cellulose (20.3 x 28.5 cm) (Whatmann 50, Maidstone, UK) was incubated with Fmoc-b-alanine-OH activated with DIC and NMI in DMF (> 3h). After some washing steps with DMF (5 x 3 min) the Fmoc-deprotection was performed using 20% piperidin in DMF (1 x 20 min), Thereafter, the membrane was washed with DMF (5 x 3 min), ethanol (3 x 3 min), and diethylether (2 x 3 min) for air-drying.

The conditions for loading of the N-Cape membrane are given in K. Licha, S. Bhargava, C. Rheinl_nder, A. Becker, J. Schneider-Mergener, R. Volkmer-Engert, Tetrahedron Lett. 2000, 41, 1711-1715 Peptide array synthesis: Overlapping peptide sequences (7-, 12- and 24-mer) with a shift of three amino acids) of Api g 1.0101 were prepared by a MultiPep SPOT-robot (INTAVIS Bioanalytical Instruments AG, Köln, Germany). Array design was performed with the aid of the in-house software LISA 1.83. Amino acids were used as Fmoc-protected Opfp-esters with the following side protection groups: tert-butyl ester (OtBu) for E and D, tert-butyl ether (tBu) for S, T and Y, tert-butoxycarbonyl (Boc) for K and W, triphenylmethyl (Trt) for C, N, Q and H and 2,2,4,6,7-pentamethyldihydrobenzofuran-5-sulfonyl (Pbf) for R. For standard SPOT-synthesis, double couplings of 0.3 M solutions of amino acids in 1-methyl-2-pyrollidone (NMP; Fluka, Steinheim, Germany) were applied. After the final Fmoc-deprotection step and washing procedure, cleavage of the side chain protecting groups was performed according to our standard two-step deprotection protocol.

Human IgE antibody binding studies: The spotted peptide arrays were prewashed with ethanol (1 × 10 min), washed with Tris-buffered saline (TBS), pH 8.0 (3 × 10 min), and blocked for 2 h at room temperature with blocking buffer. The membranes were incubated with the patients' sera (dilution 1:5) in blocking buffer overnight at 4°C, washed with TBS, followed by a second incubation with anti-human IgE antibody (ε-chain specific) conjugated with horse radish peroxidase (HRP) (A 9667, Sigma-Aldrich, St. Louis, USA) in blocking buffer for 2 h at room temperature. To remove excess antibody the membrane was washed with TBS (3x10 min). Detection was performed with Uptilight HRP blot chemiluminescent substrate (Uptima, Interchim, Montiuçon Cedex, France) with an exposure time of 2 min. The signal intensities were recorded as Biomedical Light Units (BLU) by a Lumi Imager™ (Boehringer Mannheim GmbH, Mannheim, Germany) using the LumiAnalyst software.

To evaluate the best assay parameters, various blocking reagents, antibodies and incubation parameters were investigated. For experiments verifying different blocking buffer solutions the following blocking reagents were used: (1) blocking buffer (blocking concentrate (Sigma-Genosys, Cambridge, UK)) diluted 1:10 in TBS (pH 8.0), containing 5% sucrose), (2) Roti®-ImmunoBlock (blocking concentrate, made on a polymer basis without detergent (Carl Roth, Karlsruhe, Germany) was prepared according the manufacturer's instructions); (3) milk-based blocking buffer (2.5% (w/v) skimmed dry milk in TBS (pH 8.0), containing 0.05% (v/v) Tween 20 and 5% (w/v) sucrose); (4) BSA-based blocking buffer (5% (w/v) BSA (A 2153, Sigma-Aldrich, Steinheim, Germany) in TBS (pH 8.0) containing 0.05% (v/v) Tween 20); (5) Tween 20 buffer (TBS (pH 8.0), containing 0.2% (v/v) Tween 20 (for blocking procedure) or 0.05% (v/v) Tween 20 (for antibody incubation). Blocking procedure was performed for 2 h at room temperature or for 16 h at 4°C, respectively. To evaluate the best suitable system for detecting peptide-bound IgE antibodies a direct and the indirect detection method was performed. For the direct method two different polyclonal anti-human IgE antibodies, both specific for the ε-heavy chain and labeled with HRP, were used (P 0295; Dako, Glostrup, Denmark and A 9667, Sigma-Aldrich, Saint Louis, USA). For the indirect method, either a monoclonal unlabeled mouse anti-human IgE antibody (I 6510, Sigma-Aldrich, Saint Louis, USA) recognized by a HRP-labeled anti-mouse IgG antibody (A 5906, Sigma-Aldrich, Saint Louis, USA) or a biotinylated anti-human IgE antibody, specific for the ε-heavy chain (BA-3040, Vector laboratories, Burlingame, USA), recognized by streptavidin-HRP (S 5512, Sigma-Aldrich, Saint Louis, USA) were used. At first, various peptide arrays were incubated with detection antibodies only (with and without blocking reagent), to identify cross-reactivity between detection antibodies and cellulose-bound peptides. Additionally, peptide arrays were incubated with unbound HRP and streptavidin-bound HRP to identify false positive signals caused by binding of HRP to the peptides. For comparison, peptide-arrays were incubated with sera of celery allergic and healthy patients, using the above-mentioned detection systems. For optimal signal detection, serum incubations were performed with different serum dilutions (1:1, 1:5, 1:10 and 1:20), various incubation times (1 h, 2 h and overnight) and temperatures (room temperature, 4°C and 37°C).

Measurement of spot signal intensities: Analysis and measurement of spot signal intensities were executed with the LumiAnalyst software, version 3.1 (Boehringer Mannheim GmbH, Mannheim, Germany). The spot signal was calculated from a circular region around the spot center detected on the image. To guarantee uniform spot signal assessment, and reduce signal variations caused by the background, we choose the background correction method in the LumiAnalyst software. This method allows local background correction when calculating the BLU of each spot. The background values of each spot were determined from BLU values around the spot with a safety margin and normalized to the area of the spot. Comparing replicas of several membranes, incubated under identical conditions, we found signal intensity deviations of a factor of 11. However, the relation of the signals was constant for all peptides. Therefore, to enable comparison of signal intensities (SI) from different membranes, SIs were first normalized using the following equation:

$$SI^{norm} = \frac{SI^{measured} - SI^{min}}{SI^{max} - SI^{min}}$$

An IgE antibody binding spot was defined as positive if its normalized signal intensity (SInorm) was higher than the calculated cut off value of the appropriate membrane. For calculation of the cut off value of each membrane, the mean signal intensity of all spots on this membrane was used (SImean) and fitted into the following equation:

$$Cut\ off\ value = \frac{SI^{mean} - SI^{min}}{SI^{max} - SI^{min}}$$

Statistical analysis: Loading values from the modification steps of preparing the N-CAPE membrane preparation and differences in IgE-levels between the groups were evaluated by applying the Mann-Whitney U test. The Spearman rank test was used to calculate correlations between the number of recognized peptides in the N-terminal region and the C-terminal region in sera of celery allergic patients. For comparison of specific IgE binding to Api g 1.0101 derived peptides between celery allergic and healthy patients, the Wilcoxon test was used. All statistical tests were 2-sided, and P-values ≤ 0.05 were considered statistically significant. Calculations were performed with SPSS 16.0 (SPSS Inc., Chicago, IL, USA).

EP 2 402 030 A1

**Claims**

1.  A polypeptide molecule having a sequence length of ten, eleven or twelve amino acids, said polypeptide molecule having an amino acid sequence comprised as a contiguous sequence in SEQ 001, for use as a medicament.

2.  A polypeptide molecule having a sequence length of ten to fifteen amino acids, said polypeptide molecule having an amino acid sequence comprised as a contiguous sequence in SEQ 012, SEQ 013 or SEQ 014, for the treatment or prevention of celery allergy.

3.  A polypeptide molecule according to claim 1 or 2, **characterized by** a sequence length of 12 amino acids.

4.  A pharmaceutical composition comprising one or several polypeptide molecule species having a sequence length of ten to fifteen amino acids, said polypeptide molecule species having an amino acid sequence comprised as a contiguous sequence in SEQ 012, SEQ 013 or SEQ 014, for the treatment or prevention of celery allergy.

5.  A method of diagnosing celery allergy in a patient, comprising the steps of

    - providing a polypeptide molecule having a sequence length of ten to fifteen amino acids, said polypeptide molecule having an amino acid sequence comprised as a contiguous sequence in SEQ 012, SEQ 013 or SEQ 014,

- contacting said polypeptide molecule with a patient sample comprising IgE molecules ex-vivo under conditions allowing for the specific binding of IgE molecules to IgE epitopes, and
- determining whether said polypeptide molecule is specifically bound by an IgE molecule comprised in said patient sample.

6. A method according to claim 5, **characterized by** the following steps:

- the polypeptide is provided as attached to a surface, or is attached to a surface
- a patient sample is brought into contact with said surface, resulting in IgE molecules specific for said polypeptides binding to the polypeptides,
- sample components not bound to the surface are washed off by a washing step, and
- the binding of IgE molecules is determined.

7. A method according to claim 6, **characterized in that** the binding of IgE molecules is determined

- by bringing the surface in contact with a binding agent for IgE molecules, such as an IgE-specific secondary antibody, or
- by determining the binding event through a change of physical parameters effected by the binding event.

8. A means for diagnosing celery allergy, comprising polypeptide molecules having a sequence length of ten to fifteen amino acids, said polypeptide molecules having an amino acid sequence comprised as a contiguous sequence in SEQ 012, SEQ 013 or SEQ 014.

9. A means for diagnosing celery allergy according to claim 8, **characterized in that** the means comprises a surface to which said polypeptide molecules are covalently attached.

10. A means for diagnosing celery allergy according to claim 8 or 9, **characterized in that** the means is an ELISA plate.

11. A method of detecting the presence of celery allergen api 1 peptide epitopes in a sample, comprising the steps of

- providing a molecular structure capable of specifically binding a polypeptide molecule comprising an amino acid sequence comprised as a contiguous sequence in SEQ 012, SEQ 013 or SEQ 014,
- contacting a sample with said molecular structure, and
- determining whether celery allergen api 1 peptide epitopes have bound to said molecular structure.

12. A method according to claim 11, **characterized in that** the molecular structure capable of specifically binding a polypeptide molecule is an immunoglobulin molecule or an immunoglobulin variable chain molecule.

13. A means for detecting the presence of celery allergen api 1 peptide epitopes in a sample, comprising a molecular structure capable of specifically binding a polypeptide molecule having a sequence length of ten to fifteen amino acids, said polypeptide molecule having an amino acid sequence comprised as a contiguous sequence in SEQ 012, SEQ 013 or SEQ 014.

14. A means for detecting the presence of celery allergen api 1 peptide epitopes according to claim 13, **characterized in that** the molecular structure capable of specifically binding a polypeptide molecule is an immunoglobulin molecule or an immunoglobulin variable chain molecule.

Fig 1 A

A)  7-mer   12-mer   24-mer

B)  7-mer   12-mer   24-mer

Fig 1 B

A)   B)

C)  30 nmol/cm$^2$   175 nmol/cm$^2$   700 nmol/cm$^2$

Fig 1 C

A)   B)   C)   D)   E)

Celery allergic patients / Healthy subjects — peptide reactivity grid

Europäisches
Patentamt

European
Patent Office

Office européen
des brevets

# EUROPEAN SEARCH REPORT

**Application Number**

EP 10 16 8359

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BREITENDER HEIMO ET AL: "Molecular characterization of Api g 1, the major allergen of celery (Apium graveolens), and its immunological and structural relationships to a group of 17-kDa tree pollen allergens" EUROPEAN JOURNAL OF BIOCHEMISTRY, vol. 233, no. 2, 1995, pages 484-489, XP002596864 ISSN: 0014-2956 * abstract * * figure 1 * | 1-14 | INV. A61K39/35 C07K7/04 C07K7/06 C07K16/16 G01N33/68  ADD. A01H5/04 A01H5/06 |
| A,D | BOHLE BARBARA ET AL: "Bet v 1, the major birch pollen allergen, initiates sensitization to Api g 1, the major allergen in celery: Evidence at the T cell level." EUROPEAN JOURNAL OF IMMUNOLOGY, vol. 33, no. 12, December 2003 (2003-12), pages 3303-3310, XP002596865 ISSN: 0014-2980 * abstract * * page 3304, left-hand column, line 8 - line 22 * * Api g 1-specific T cell epitopes.; page 3305, paragraph 2.5 * | 1-14 | |
| | -/-- | | TECHNICAL FIELDS SEARCHED (IPC)  A61K C07K G01N |

The present search report has been drawn up for all claims

| Place of search | Date of completion of the search | Examiner |
|---|---|---|
| Munich | 17 August 2010 | Mundel, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 16 8359

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SCHIRMER ET AL: "Crystal Structure of the Major Celery Allergen Api g 1: Molecular Analysis of Cross-reactivity" JOURNAL OF MOLECULAR BIOLOGY, LONDON, GB, vol. 351, no. 5, 2 September 2005 (2005-09-02), pages 1101-1109, XP005022196 ISSN: 0022-2836 * abstract * * Comparison of putative cross-reactive epitopes with Bet v 1 residues involved in IgE recognition, inferred by mutagenesis studies.; page 1105 - page 1106 * * page 1107, left-hand column, line 9 - line 26 * | 1-14 | |
| A | EP 2 028 188 A1 (BIOMAY AG [AT]) 25 February 2009 (2009-02-25) * abstract * * paragraph [0006] * * paragraph [0012] * * paragraph [0016] * | 1-14 | |
| A,D | BOHLE ET AL: "Cooking birch pollen-related food: Divergent consequences for IgE- and T cell-mediated reactivity in vitro and in vivo" JOURNAL OF ALLERGY AND CLINICAL IMMUNOLOGY, MOSBY, INC, US LNKD-DOI:10.1016/J.JACI.2006.03.011, vol. 118, no. 1, 1 July 2006 (2006-07-01), pages 242-249, XP005610127 ISSN: 0091-6749 * abstract * * Thermal processing of food allergens reduces mediator release; page 245 * | 1-14 | TECHNICAL FIELDS SEARCHED (IPC) |

The present search report has been drawn up for all claims

| Place of search | Date of completion of the search | Examiner |
|---|---|---|
| Munich | 17 August 2010 | Mundel, Christophe |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 10 16 8359

This annex lists the patent family members relating to the patent documents cited in the above-mentioned European search report.
The members are as contained in the European Patent Office EDP file on
The European Patent Office is in no way liable for these particulars which are merely given for the purpose of information.

17-08-2010

| Patent document cited in search report | | Publication date | Patent family member(s) | | Publication date |
|---|---|---|---|---|---|
| EP 2028188 | A1 | 25-02-2009 | AU | 2008290883 A1 | 26-02-2009 |
| | | | CA | 2696691 A1 | 26-02-2009 |
| | | | EP | 2190865 A1 | 02-06-2010 |
| | | | WO | 2009024208 A1 | 26-02-2009 |

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BOHLE et al.** *Eur. J. Immunol.,* 2003, vol. 33, 3303-3310
- **BOHLE et al.** *J. (2006) Allergy Clin. Immunol.,* 2006, vol. 118, 242-249
- **HAUSMANN et al.** *The Cell: a molecular approach,* ISBN 0-87893-214-3


SEQUENCE LISTING

<110> Charité Universitätsmedizin Berlin

<120> Linear IgE peptide epitopes of celery allergen api 1

<130> ipa102ep

<160> 14

<170> PatentIn version 3.5

<210> 1
<211> 12
<212> PRT
<213> Apium graveolens

<400> 1

Val Leu Val Pro Thr Ala Asp Gly Gly Ser Ile Cys
1               5                   10

<210> 2
<211> 12
<212> PRT
<213> Apium graveolens

<400> 2

Ala Glu Lys Ile Phe Gln Gly Phe Val Ile Asp Val
1               5                   10

<210> 3
<211> 12
<212> PRT
<213> Apium graveolens

<400> 3

Ile Phe Gln Gly Phe Val Ile Asp Val Asp Thr Val
1               5                   10

<210> 4
<211> 12
<212> PRT
<213> Apium graveolens

<400> 4

Gly Phe Val Ile Asp Val Asp Thr Val Leu Pro Lys
1               5                   10

<210> 5
<211> 12
<212> PRT
<213> Apium graveolens

<400> 5

Gly Asp Gly Gly Pro Gly Thr Leu Lys Ile Ile Thr
1               5                   10

<210> 6

```
<211>   12
<212>   PRT
<213>   Apium graveolens

<400>   6

Gly Pro Gly Thr Leu Lys Ile Ile Thr Leu Pro Asp
1                   5                   10


<210>   7
<211>   12
<212>   PRT
<213>   Apium graveolens

<400>   7

Thr Leu Lys Ile Ile Thr Leu Pro Asp Gly Gly Pro
1                   5                   10


<210>   8
<211>   12
<212>   PRT
<213>   Apium graveolens

<400>   8

Ile Ile Thr Leu Pro Asp Gly Gly Pro Ile Thr Thr
1                   5                   10


<210>   9
<211>   12
<212>   PRT
<213>   Apium graveolens

<400>   9

Leu Pro Asp Gly Gly Pro Ile Thr Thr Met Thr Leu
1                   5                   10


<210>   10
<211>   12
<212>   PRT
<213>   Apium graveolens

<400>   10

Gly Gly Pro Ile Thr Thr Met Thr Leu Arg Ile Asp
1                   5                   10


<210>   11
<211>   12
<212>   PRT
<213>   Apium graveolens

<400>   11

Pro Thr Ala Asp Gly Gly Ser Ile Cys Lys Thr Thr
1                   5                   10


<210>   12
<211>   21
<212>   PRT
```

<210>    Apium graveolens

<400>    12

Ser  Val  Ser  Ala  Glu  Lys  Ile  Phe  Gln  Gly  Phe  Val  Ile  Asp  Val  Asp
1                   5                   10                  15

Thr  Val  Leu  Pro  Lys
                    20


<210>    13
<211>    27
<212>    PRT
<213>    Apium graveolens

<400>    13

Gly  Asp  Gly  Gly  Pro  Gly  Thr  Leu  Lys  Ile  Ile  Thr  Leu  Pro  Asp  Gly
1                   5                   10                  15

Gly  Pro  Ile  Thr  Thr  Met  Thr  Leu  Arg  Ile  Asp
                    20                  25


<210>    14
<211>    21
<212>    PRT
<213>    Apium graveolens

<400>    14

Asn  His  Val  Val  Leu  Val  Pro  Thr  Ala  Asp  Gly  Gly  Ser  Ile  Cys  Lys
1                   5                   10                  15

Thr  Thr  Ala  Ile  Phe
                    20